# EUROPÄISCHE PATENTSCHRIFT

(11) **EP 0 575 917 B1**
(45) Veröffentlichungstag und Bekanntmachung des Hinweises auf die Patenterteilung: **22.03.2000**
(21) Anmeldenummer: 93109796.8
(22) Anmeldetag: 18.06.1993
(51) Int. Cl.: A61B 5/15, A61M 5/315

(54) **Blutentnahme-Vorrichtung**
Blood sampling device
Dispositif de prise de sang

(30) Priorität: 22.06.1992 DE 4220301
(43) Veröffentlichungstag der Anmeldung: 29.12.1993
(73) Patentinhaber: pvb medizintechnik gmbh & co. kg, 85614 Kirchseeon/Eglharting (DE)
(72) Erfinder: Von Berg, Peter, Tiburon, CA 94920 (US)
(74) Vertreter: von Bülow, Tam, Dr.

(56) Entgegenhaltungen:
- EP-A- 0 086 456
- EP-A- 0 178 568
- EP-A- 0 301 913
- US-A- 4 215 701

## Beschreibung

Die Erfindung bezieht sich auf eine Blutentnahme-Vorrichtung. Eine Vorrichtung gemäß dem Oberbegriff des Patentanspruches 1 ist aus dem Dokument US-A-4 215 701 bekannt. Weitere Vorrichtungen werden in den Dokumenten EP-A2-0 302 752 und WO 88/01846 beschrieben. In Fällen, bei denen ein Infusionsschlauch oder ein Katheter in eine Vene oder Arterie eines Patienten eingesetzt ist, beispielsweise für eine Tropfinfusion und/oder eine permanente, direkte Blutdruckmessung wird, wenn für Untersuchungszwecke eine Blutprobe entnommen werden soll, diese an einer von außen zugänglichen Stelle des Infusionsschlauches entnommen. Hierzu sehen die oben genannte Vorrichtungen Entnahmestellen vor, die mit einem Strömungskanal des Infusionssystems in Verbindung stehen und durch eine Kolbenspritze von außen zugänglich sind.

Bevor eine Blutentnahme aus diesem System vorgenommen werden kann, muß zunächst dafür gesorgt werden, daß sich im Bereich der Entnahmestelle nur unverdünntes Blut des Patienten und keine Anteile von Infusionslösungen oder Verdünnungsmitteln befinden, die bei der direkten Blutdruckmessung zum Verhindern der Koagulation des Blutes zugeführt werden. Zu diesem Zwecke sieht die WO 88/01846 zwei Entnahmestellen vor. Die näher zum Patienten liegende (vordere) Entnahmestelle dient der eigentlichen Entnahme der Blutprobe. Die weiter vom Patienten entfernt liegende (hintere) Entnahmestelle dient dazu, vorübergehend Infusionslösungen aus dem System zu entfernen, damit an der vorderen Entnahmestelle nur noch unverdünntes Blut vorhanden ist. Nach der Blutentnahme an der vorderen Entnahmestelle wird dann die an der hinteren Entnahmestelle entnommene Menge aus Infusionslösung und Blut wieder dem System zugeführt.

Bei der WO 88/01846 erfolgt die Entnahme und das Wiederzuführen an der hinteren Entnahmestelle mittels einer herkömmlichen Spritze, deren Nadel einen ansonsten dichten Verschlußstopfen durchdringt. Dies setzt aber ein aufwendiges Hantieren voraus, bringt die Gefahr mit sich, daß durch die Spritzennadel Verunreinigungen, Keime und sonstige Krankheitserreger dem System zugeführt werden und bringt für das Klinikpersonal die Gefahr mit sich, daß es sich beim Entfernen der Nadel daran verletzt und infiziert, was in der Vergangenheit schon zu einer großen Anzahl von Aids-Infektionen bei Klinikpersonal geführt hat.

Es wurde daher schon vorgeschlagen, insoweit ein geschlossenes System zu verwenden, als die hintere Entnahmestelle als ein permanent in das Infusions- oder Kathetersystem integrierter Zwischenspeicher mit einer Kolben-/Zylinderanordnung ausgestaltet ist, die von außen nicht mehr zugänglich ist (vgl. Firmenprospekt der Firma Baxter mit dem Titel "New Vamp; a closed system for easier, safer blood sampling from invasive lines"; sowie Firmenprospekt der Firma Pfrimmer Viggo GmbH & Co. KG mit dem Titel "Saw draw; geschlossenes Blutentnahmesystem mit Statham-Einmal-Druckwandler DTX/plus).

Bei diesen bekannten Blutentnahmevorrichtungen tritt jedoch noch das Problem auf, daß der Zwischenspeicher bei Reinfusion des heparinisierten Patientenblutes nicht vollständig entleert wird, so daß dort verbleibende Blutreste koagulieren. Bei mehrmaliger Durchführung der oben genannten Vorgänge kann es somit vorkommen, daß koagulierte Blutreste aus dem Zwischenspeicher wieder zurück in den Blutkreislauf des Patienten gelangen und ihn hochgradig gefährden.

Aufgabe der Erfindung ist es daher, die bekannten Blutentnahmevorrichtungen dahingehend zu verbessern, daß die bei der Blutentnahme in einem Reservoir zwischengespeicherten Mengen von mit Infusionslösungen vermischtem Blut vollständig dem Patienten reinfudiert werden und somit der Zwischenspeicher vollständig entleert wird.

Diese Aufgabe wird durch die im Kennzeichenteil des Patentanspruches 1 angegebenen Merkmale gelöst. Vorteilhafte Ausgestaltungen und Weiterbildungen der Erfindung sind den Unteransprüchen zu entnehmen.

Mit der Vorrichtung nach der Erfindung wird erreicht, daß beim Entleeren der Kolben-/Zylinderanordnung zunächst die radial äußeren Abschnitte der Kolbenspitze mit den entsprechenden Abschnitten des konischen Endes des Zylinderraumes in Berührung kommen und erst beim weiteren Niederdrücken des Kolbens auch die weiter radial innenliegenden Abschnitte. Damit werden auch kleinste Reste von Blut und Infusionslösung aus dem Zylinderraum herausgedrückt.

Mit Anspruch 2 wird erreicht, daß auch die Strömungsverbindung zwischen dem Infusionskanal und dem Zylinderraum vollständig entleert wird.

Mit Anspruch 3 wird eine konstruktiv günstige und einfach herzustellende Lösung realisiert, bei der die erforderliche Flexibilität für die Verformung der Spitze der Kolbendichtung nicht nur durch die Eigenelastizität des Materiales sondern auch durch die Form hervorgerufen wird.

Mit den Ansprüchen 4 und 5 wird die zum vollständigen Entleeren des Zylinderraumes erforderliche Verformung der Kolbendichtung gezielt beeinflußt und ein Verklemmen oder Verkanten - und damit eine unerwünschte Verformung der Kolbendichtung vermieden. Letzterem dienen auch die Merkmale des Anspruches 6.

Im folgenden wird die Erfindung anhand eines Ausführungsbeispieles im Zusammenhang mit der Zeichnung ausführlicher erläutert. Es zeigt:
- Fig. 1: einen Querschnitt der Blutentnahmevorrichtung nach der Erfindung;
- Fig. 2: einen um 90° gedrehten Querschnitt der Vorrichtung gemäß Fig. 1 (bei der Kolbenanordnung nicht geschnitten ist); und
- Fig. 3: einen Querschnitt einer im Ausführungsbeispiel der Fig. 1 und 2 verwendeten Kolbendichtung.

Gleiche Bezugszeichen in den einzelnen Figuren bezeichnen gleiche Teile.

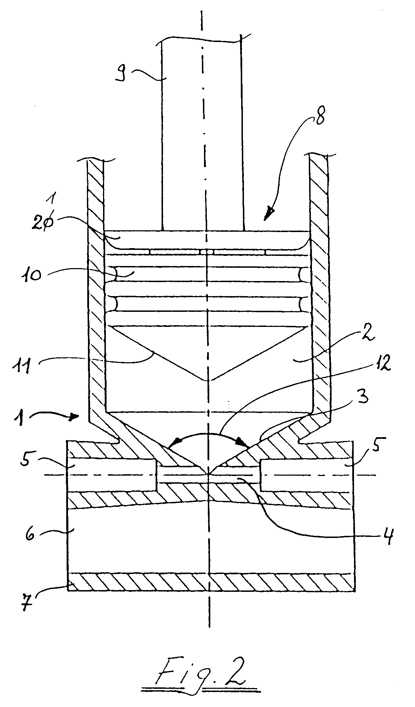

Die Vorrichtung besitzt eine Gehäuse 1 mit einer zylindrischen Kammer 2, deren mit einem Durchflußkanal 4 in Verbindung stehende Spitze 3 konisch spitz zulaufend ausgebildet ist, d.h. die Form eines Kegelstumpfes hat. Wie aus Fig. 2 zu erkennen, ist der Durchflußkanal 4 beidseitig mit Anschlüssen 5 versehen, in welche mit herkömmlichen Kupplungen Leitungsabschnitte eines Infusionsschlauches eingesetzt werden können. Anschließend an die konische Spitze 3 des Zylinderraumes 2 folgen zwei senkrecht zueinander stehende Stege 6 und 7, die zur Halterung der Vorrichtung dienen.

Im Zylinderraum 2 ist ein Kolben 8 verschieblich geführt, der über eine Kolbenstange 9 betätigbar ist. Der Kolben 8 ist von einer flexiblen Kolbendichtung 10 umhüllt, die ebenfalls eine konische bzw. kegelstumpfförmige Spitze 11 aufweist.

Der Konuswinkel 12 der Spitze 3 des Zylinderraumes 2 ist kleiner als der Konuswinkel 13 der konischen Spitze 11 der Kolbendichtung 10. Mit anderen Worten ist der Konus der Kolbenspitze flacher als der der Zylinderspitze. Dies hat zur Folge, daß beim Niederdrucken des Kolbens 8 zunächst der radial äußere Rand der konischen Spitze 11 mit dem entsprechenden Rand der Spitze 3 des Zylinderraumes in Berührung kommt. Erst beim weiteren Niederdrücken des Kolbens verformt sich die konische Spitze 11 der Kolbendichtung derart, daß zunehmend von außen nach innen die Kolbendichtung an der konischen Spitze 3 des Zylinderraumes 2 zur Anlage kommt. Damit werden Flüssigkeitsreste im Zylinderraum von außen nach innen in Richtung auf den Durchflußkanal 4 gedrückt.

Ist der Kolben 8 in seine unterste Grenzstellung gefahren, so hat sich die Kolbendichtung 10 soweit verformt, daß die konischen Spitzen 11 und 3 des Kolbens 8 bzw. des Zylinderraumes 3 vollflächig aneinander anliegen und der dem Durchflußkanal 4 unmittelbar gegenüberliegende Abschnitt der Kolbendichtung in den Durchflußkanal hineinragt. Damit sind der Zylinderraum 2 und auch die Verbindung zwischen dem Durchflußkanal 4 und dem Zylinderraum vollständig entleert. Hierzu steht der Durchflußkanal 4 auch unmittelbar, d.h. ohne einen zwischengeschalteten Verbindungsabschnitt mit dem Zylinderraum 2 in Verbindung. Wie besonders gut aus Figur 2 zu erkennen ist, schneidet eine von der Oberkante des Durchflußkanales 2 gebildete Linie einen Teil der Spitze des konischen Abschnittes 3.

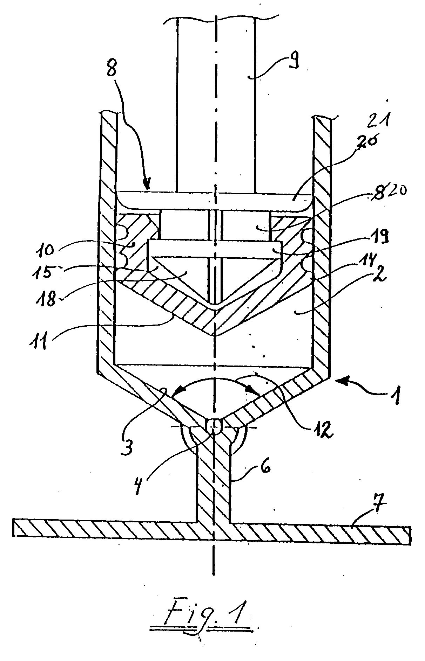

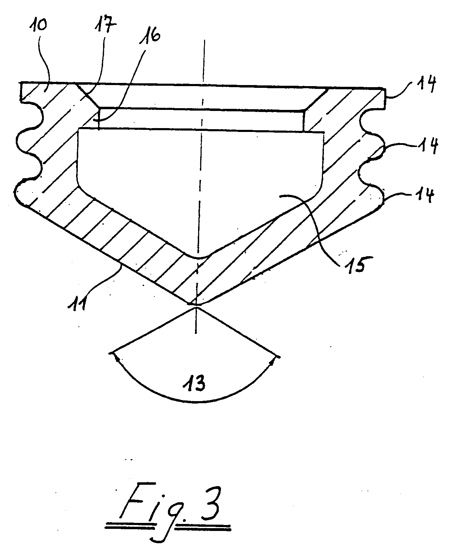

Der Kolben 8 weist ausgehend von seinem freien Ende einen konischen Abschnitt 18, einen zylindrischen Rastvorsprung 19, einen zylindrischen Abschnitt 20 und einen radial vorstehenden Bund 21 auf. Die Abschnitte 18 und 20 sind hier durch zwei sich rechtwinklig schneidende Stege gebildet und sind daher nicht rotationssymmetrisch. Der Durchmesser des Bundes 21 entspricht im wesentlichen dem Innendurchmesser des Zylinderraumes 2. Die Kolbendichtung 10 übergreift die genannten Abschnitte 18, 19 und 20 des Kolbens und ist - wie am besten aus Fig. 3 zu erkennen - wie folgt geformt: An die konische Spitze 11 schließt sich ein im wesentlichen zylindrischer Abschnitt an, der an seiner Außenkontur mehrere Dichtlippen 14 aufweist, die an der Innenwand des Zylinderraumes anliegen. Die Kolbendichtung 10 besitzt einen hohlen Innenraum 15, dessen Wände im wesentlichen parallel zu den Außenwänden verlaufen. Ein radial nach innen ragender Rastvorsprung übergreift den Bund 19 des Kolbens 8, womit die Kolbendichtung 10 an dem Kolben gehalten ist. Zur Montage der Kolbendichtung weist diese anschließend an den Bund 16 noch eine Fase 17 auf. Der Bund 16 liegt - wie aus Fig. 1 zu erkennen - bei montierter Kolbendichtung zwischen dem Rastvorsprung 19 und dem Bund 21 des Kolbens 8. Dabei ist - wie ebenfalls aus Fig. 1 zu ersehen - ein gewisses Spiel vorhanden, innerhalb dessen Grenzen eine Relativbewegung zwischen dem Kolben 8 und der Kolbendichtung 10 möglich ist. In der in Fig. 1 dargestellten Position wird der Kolben 8 herausgezogen. Der Rastvorsprung 19 liegt an dem Bund 16 an, während zwischen der Oberseite der Kolbendichtung und dem Bund 21 ein Freiraum ist und auch der konische Abschnitt 18 des Kolbens keine Berührung mit den Innenwänden der Kolbendichtung hat.

Weiter ist aus Fig. 1 zu erkennen, daß der Konuswinkel des konischen Abschnittes 18 kleiner ist als der Konuswinkel (13 in Fig. 3) der Kolbendichtung.

Wird nun der Kolben nach unten gedrückt, so durchläuft der Kolben zunächst das Spiel und der Bund 21 drückt gegen die Oberseite der Kolbendichtung 10. Kommt dann der radial äußere Rand der Kolbendichtung 10 mit dem entsprechenden Rand der konischen Spitze 3 des Zylinderraumes 2 in Berührung, so verformt sich mit zunehmendem Druck der Bund 16 und die Spitze des konischen Abschnittes 18 drückt gegen die Innenwand der konischen Spitze 11 der Kolbendichtung 10, wodurch deren Konuswinkel durch Verformung spitzer wird. Weiter wird dadurch sichergestellt, daß die dem Durchflußkanal 4 gegenüberliegende Spitze der Kolbendichtung in den Durchflußkanal 4 hineingedrückt wird.

## Patentansprüche

1. Blutentnahme-Vorrichtung mit einer Kolben-/Zylinderanordnung, die mit einem Durchflußkanal (4) in Verbindung steht, bei der die Kolbenanordnung eine elastische Kolbenspitze (11) aufweist, die konisch geformt ist, und bei der die Zylinderanordnung einen Zylinderraum (2) bildet, der eine konische Spitze (3) aufweist, dadurch gekennzeichnet,
daß der Konuswinkel (13) der Kolbenspitze (11) größer ist als der Konuswinkel (12) des Zylinderraumes (2) und
daß die Kolbenspitze (11) soweit elastisch ist, daß beim Andrücken der Kolbenspitze (11) gegen die konische Spitze (3) des Zylinderraumes (2) die Differenz der Konuswinkel (12, 13) eliminiert wird.

2. Blutentnahme-Vorrichtung nach Anspruch 1, dadurch gekennzeichnet, daß der Durchflußkanal (4) zum Zylinderraum (2) hin offen ist und daß bei niedergedrücktem Kolben (8) ein Abschnitt der Kolbenspitze (11) in den Durchflußkanal (4) hineinragt.

3. Blutentnahme-Vorrichtung nach Anspruch 1 oder 2, dadurch gekennzeichnet, daß die Kolbenspitze durch eine flexible Kolbendichtung (10) gebildet ist, daß die Kolbendichtung (10) einen radial nach innen ragenden Bund (16) aufweist, an den sich ein Hohlraum (15) im Inneren der Kolbendichtung (10) anschließt, daß der Kolben (8) mit einem konischen Abschnitt in den Hohlraum (15) hineinragt, wobei der Konuswinkel dieses Abschnittes (18) kleiner ist als der Konuswinkel (13) der Kolbenspitze (11) und daß der Bund (16) der Kolbendichtung (10) zwischen zwei radial vorspringenden Schultern (19, 21) gehalten ist.

4. Blutentnahme-Vorrichtung nach Anspruch 3, dadurch gekennzeichnet, daß zwischen den beiden Schultern (19, 21) des Kolbens (8) und dem Bund (16) der Kolbendichtung ein Spiel vorhanden ist.

5. Blutentnahme-Vorrichtung nach Anspruch 3 oder 4, dadurch gekennzeichnet, daß zwischen dem freien Ende des konischen Abschnittes (18) des Kolbens (8) und der Innenwandung des Innenraumes (15) der Kolbendichtung (10) ein Spiel vorhanden ist.

6. Blutentnahme-Vorrichtung nach den Ansprüchen 3 bis 5, dadurch gekennzeichnet, daß die Kolbendichtung (10) radial vorstehende Dichtlippen (14) aufweist, die mit der Innenwand des Zylinderraumes (2) in Berührung stehen.

## Claims

1. Blood sampling device comprising a piston/cylinder arrangement, which communicates with a flow through channel (4),
wherein the piston means comprises an elastic piston having an elastic apex (11), which is conically shaped and wherein the cylinder means constitutes a compression volume (2) comprising a conical apex (3), characterized in that
the cone angle (13) of the apex (11) of the piston is larger than the cone angle (12) of the compression volume (2) and
that the apex (11) of the piston is elastic insofar that the difference between the cone angles (12, 13) is eliminated when the apex (11) of the piston is pushed against the conical apex (3) of the compression volume (2).

2. Blood sampling device according to claim 1, characterized in that flow through channel (4) is opened in the direction of compression volume (2) and that a portion of the apex (11) of the piston projects into the flow through channel (4) when piston (8) is pushed down.

3. Blood sampling device according to claim 1 or 2, characterized in that the apex of the piston is constituted by a flexible piston sealing (10), that piston sealing (10) comprises a collar (16) radially projecting inwards adjoining a hollow space (15) in the interior of piston sealing (10), that the conical portion of piston (8) projects into the hollow space (15) wherein the cone angle of this portion (18) is smaller than the cone angle (13) of the apex (11) of the piston and that collar (16) of the piston sealing (10) is fixed between two radially projecting shoulders (19, 21).

4. Blood sampling device according to claim 3, characterized in that a clearance is provided between the two shoulders (19, 21) of piston (8) and collar (16) of the piston sealing.

5. Blood sampling device according to claim 3 or 4, characterized in that a clearance is provided between the free end of conical portion (18) of piston (8) and the inner wall of the interior space (15) of piston sealing (10).

6. Blood sampling device according to one of the claims 3 to 5, characterized in that the piston sealing (10) comprises radially projecting packing washers (14), which are in contact with the inner wall of compression volume (2).

## Revendications

1. Dispositif de prise de sang avec un agencement de piston/cylindre qui communique avec un canal de passage (4), l'agencement de piston présentant une pointe de piston élastique (11) qui a une forme conique et l'agencement de cylindre formant un espace cylindrique (2) qui présente une pointe conique (3), caractérisé
en ce que l'angle conique (13) de la pointe de piston (11) est plus grand que l'angle conique (12) de l'espace cylindrique (2) et
en ce que la pointe de piston (11) est élastique dans une mesure telle qu'en cas de pression de la pointe de piston (11) contre la pointe conique (3) de l'espace cylindrique (2), la différence de l'angle conique (12, 13) est éliminée.

2. Dispositif de prise de sang selon la revendication 1, caractérisé en ce que le canal de passage (4) est ouvert sur l'espace cylindrique (2) et en ce que le piston (8) étant abaissé, une partie de la pointe de piston (11) pénètre dans le canal de passage (4).

3. Dispositif de prise de sang selon la revendication 1 ou 2, caractérisé en ce que la pointe de piston est constituée d'une garniture de piston (10) flexible, en ce que la garniture de piston (10) présente une embase (16) qui dépasse vers l'intérieur dans le sens radial et à laquelle se rattache une cavité (15) à l'intérieur de la garniture de piston (10), en ce que le piston (8) dépasse dans la cavité (15) avec une partie conique, l'angle conique de cette partie (18) étant plus petit que l'angle conique (13) de la pointe de piston (11), et en ce que l'embase (16) de la garniture de piston (10) est maintenue entre deux épaulements (19, 21) faisant saillie dans le sens radial.

4. Dispositif de prise de sang selon la revendication 3, caractérisé en ce qu'il y a un jeu entre les deux épaulements (19, 21) du piston (8) et l'embase (16) de la garniture de piston.

5. Dispositif de prise de sang selon la revendication 3 ou 4, caractérisé en ce qu'il y a un jeu entre l'extrémité libre de la partie conique (18) du piston (8) et la paroi intérieure de l'espace intérieur (15) de la garniture de piston (10).

6. Dispositif de prise de sang selon les revendications 3 à 5, caractérisé en ce que la garniture de piston (10) présente des lèvres d'étanchéité (14) qui font saillie dans le sens radial et qui sont en contact avec la paroi intérieure de l'espace cylindrique (2).
